(19) Europäisches Patentamt
European Patent Office
Office européen des brevets

(11) Veröffentlichungsnummer: **0 196 522**
**B1**

(12) # EUROPÄISCHE PATENTSCHRIFT

(45) Veröffentlichungstag der Patentschrift: **12.07.89**

(51) Int. Cl.⁴: **C 07 D 213/73**

(21) Anmeldenummer: **86103458.5**

(22) Anmeldetag: **14.03.86**

(54) **2-Amino-4-trichlormethyl-pyridin, Verfahren zur seiner Herstellung und seine Verwendung als Nitrifikationshemmer.**

(30) Priorität: **19.03.85 DE 3509860**

(43) Veröffentlichungstag der Anmeldung: **08.10.86 Patentblatt 86/41**

(45) Bekanntmachung des Hinweises auf die Patenterteilung: **12.07.89 Patentblatt 89/28**

(84) Benannte Vertragsstaaten: **BE CH DE FR GB IT LI NL SE**

(56) Entgegenhaltungen:
**EP-A-0 001 887**
**US-A-3 787 420**
**US-A-3 799 935**

(73) Patentinhaber: **BASF Aktiengesellschaft, Carl-Bosch- Strasse 38, D-6700 Ludwigshafen (DE)**

(72) Erfinder: **Hagen, Helmut, Dr., Max- Slevogt-Strasse 17 e, D-6710 Frankenthal (DE)**
Erfinder: **Ziegler, Hans, Dr., Am Speyerweg 48, D-6704 Mutterstadt (DE)**
Erfinder: **Kohler, Rolf- Dieter, Dr., Amselweg 3, D-6803 Edingen- Neckarhausen (DE)**
Erfinder: **Pommer, Ernst- Heinrich, Dr., Berliner Platz 7, D-6703 Limburgerhof (DE)**
Erfinder: **Dressel, Juergen, Dr., Medenheimer Strasse 18, D-6708 Neuhofen (DE)**

**EP 0 196 522 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

2-Chlor-6-trichlormethylpyridin ist als Nitrifikationshemmer bekannt; die allgemeine Bezeichnung des Wirkstoffes ist Nitrapyrin. Seine Herstellung und Wirkung ist z. B. in der US-PS-3 135 594 beschrieben.

Die Anwendbarkeit dieser Verbindung ist durch ihre physikalischen und chemischen Eigenschaften eingeschränkt; sie ist nämlich, bedingt durch den niedrigen Dampfdruck leicht flüchtig, was den Einsatz als nitrifikationshemmendes Mittel erschwert.

Aminohalogenpyridine sind ebenfalls als Stoffe bekannt. Ihnen wird fungizide, bakterizide und insektizide Wirkung zugeschrieben, jedoch ist kein Stoff dieser Gruppe, die z. B. in der US-PS-3 799 935 beschrieben ist, zu praktischer Bedeutung gelangt. Trifluormethylpyridinderivate mit herbiziden, fungiziden, bakteriziden Eigenschaften sowie Wirkung gegenüber Milben und parasitären Würmern sind aus US-PS-3 787 420 bekannt.

Es wurde nun gefunden, daß 2-Amino-4-trichlormethylpyridin der Formel I

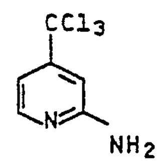

(I)

bei sehr guter Wirksamkeit, bedingt durch seine physikalischen Eigenschaften und seine großere Stabilität, eine erweiterte und sichere Anwendbarkeit besitzt.

Man kann 2-Amino-4-trichlormethyl-pyridin nach entsprechendem Schutz der Aminogruppe aus 2-Amino-4-picolin gewinnen. Der Schutz der Aminogruppe kann z. B. durch Umsetzung mit Phthalsäureanhydrid bewirkt werden. Die Halogenierung des 4-Methyl-2-phthalimido-pyridins mit Chlor zu 2-Phthalimido-4-trichlormethyl-pyridin kann ohne Isolierung dieser Verbindung in dem Reaktionsgemisch vorgenommen werden; man spaltet anschließend die Schutzgruppe durch Verseifen wieder ab, wobei sich Ethanolamin bewährt hat.

Für das Einführen der Schutzgruppe verwendet man zweckmäßig ein Lösungsmittel; es kommen z. B. Aromaten wie Benzol, Toluol, Xylol; aliphatische Kohlenwasserstoffe sowie substituierte Aromaten wie Nitrobenzol, Dichlorbenzol oder sonstige halogenierte Kohlenwasserstoffe wie Tetrachlorethan in Frage. Das entstehende Wasser wird zweckmäßigerweise azeotrop entfernt. Die Umsetzung verläuft zwischen 50 und 250° C, bevorzugt zwischen 100 bis 180° C. Soll die Halogenierung ohne Isolierung des Zwischenproduktes durchgeführt werden, so setzt man zweckmäßigerweise von vornherein ein für die Halogenierung geeignetes Lösungsmittel ein.

Bevorzugt sind Chlorbenzol, Dichlorbenzol, aber auch z. B. Nitrobenzol. Zweckmäßig wird ein Radikalbildner, wie Azodiisobuttersäurenitril in einer Menge von 0,1 % bis 10 %, vorzugsweise 0,5 % bis 1 %, bezogen auf die zu chlorierende Verbindung zugegeben. Wie üblich kann die Umsetzung auch mit Hilfe einer UV-Lichtquelle beschleunigt werden. Der Temperaturbereich für die Chlorierung liegt bei 50 bis 250° C, vorzugsweise bei 120 bis 160° C.

Die Verseifung wird zweckmäßig nach der in der DE-OS-3 319 650 beschriebenen Methode vorgenommen.

Für die Verseifung empfiehlt sich als Lösungsmittel ein Überschuß an Ethanolamin, Wasser, ein Alkohol wie Ethanol, Propanol, Methylglykol, ein Keton wie Aceton, ein chlorierter Kohlenwasserstoff wie Chloroform oder ein Ether wie Diethylether. Bevorzugt sind ein Überschuß an Ethanolamin, Ethanol und Propanol. Der bevorzugte Temperaturbereich für die Verseifung liegt bei -10° C bis 30° C.

**Beispiel**

108,1 g 2-Amino-4-picolin wurden in 1 kg 1,2-Dichlorbenzol mit 148 g Phthalsäureanhydrid 2 Stunden unter Rückfluß an einem auf über 100° C gehaltenen Kühler auf 170° C erwärmt und das gebildete Wasser abdestilliert. Die Mischung wurde auf 140° C abgekühlt und mit 1 g Azodiisobuttersäurenitril versetzt. Bei 140° C wurden 400 g Chlor eingeleitet, danach wurde abgekühlt, ein Teil des Lösungsmittels i.V. entfernt und der Rückstand mit Petrolether versetzt. Der Niederschlag wurde abgetrennt, mit Petrolether gewaschen und getrocknet. Es wurden 320 g 2-Phthalimido-4-trichlormethylpyridin (entsprechend 94 % der berechneten Menge; Schmp.: 143° C) erhalten.

Von dem Vorprodukt wurden 105 g bei 20 bis 30° C in 600 g Ethanolamin eingetragen. Nach 30 Minuten wurde in 2 l Eiswasser gegeben, der Niederschlag abgetrennt mit Wasser gewaschen und getrocknet. Es wurden 122 g 2-Amino-4-trichlormethylpyridin (entsprechend 96 %; Schmp.: 114° C) erhalten.

**Anwendungsbeispiel**

Zu 200 g eines nicht sterilisierten, dem Freiland entnommenen lehmigen Sandbodens, dessen Feuchtigkeitsgehalt auf 50 % der maximalen Wasserkapazität eingestellt war, wurden 220 mg Ammoniumsulfat gegeben und mit dem Boden gründlich vermischt. Danach wurden die Wirkstoffe, in 0,2 ml Aceton gelöst, in Mengen von jeweils 2, 1, 0,5 und 0,25 ppm, bezogen auf feuchten Sandboden, zugesetzt. Nach sorgfältiger Durchmischung wurden die Bodenproben nach Verdunsten des Acetons in mit Aluminiumfolie zur Vermeidung von Wasserverlusten bedeckten 1-Liter-Gläsern zusammen mit den Kontrollen ohne Wirkstoffzusatz über einen Zeitraum

von 28 Tagen bei 21°C bebrütet (Nach diesem Zeitraum enthält eine Bodenprobe mit normaler Sodengare im allgemeinen keine nachweisbaren Mengen von Ammoniumstickstoff mehr).

Danach wurden jeweils 2,5 g der Bodenproben in 100-ml-Erlenmeyerkolben eingefüllt und 22,5 ml einer 0,1n Kaliumsulfat-Lösung zugesetzt. Nach 30minütigem Schütteln wurde abfiltriert und jeweils 2,5 ml der Bodenauszüge wurden mit 1625 ml destilliertem Wasser vermischt. Anschließend wurden zum Nachweis der im Bodenauszug noch vorhandenen Ammoniumionen 1,25 ml Neßler-Reagenz hinzugefügt und gründlich geschüttelt. Die Farbveränderungen wurden dann photometrisch bei einer Wellenlänge von 420 ηm gemessen. Anhand von Standardkurven, die durch Messung von Lösungen mit bekannten Ammoniumsulfat-Gehalten ermitteln worden waren, wurden die noch in den Bodenproben vorhandenen Ammoniumsulfat-Mengen bestimmt. Die prozentuale Hemmung der Nitrifikation in den behandelten Bodenproben wurde im Vergleich mit den unbehandelten Bodenproben (nur Ammoniumsulfatzusatz) nach folgender Formel berechnet:

... % Hemmung der Nitrifikation = $\frac{a-b}{a} \cdot 100$

a = Nitrifikationsrate von Ammoniumsulfat (mit 100 % bzw. 1.0 angenommen)
b = Nitrifikationsrate von Ammoniumsulfat + Nitrifikationshemmer

| Wirkstoff-Zusatz ... ppm | ... % Hemmung der Nitrifikation 4 Wochen nach Zugabe von 2-Amino-4-trichlormethylpyridin zum Boden |
|---|---|
| 2 | 100 |
| 1 | 97 |
| 0,5 | 94 |
| 0,25 | 48 |

**Patentansprüche**

1. 2-Amino-4-trichlormethylpyridin der Formel I

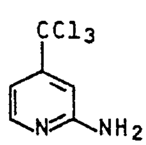

(I)

2. Verfahren zur Herstellung von 2-Amino-4-trichlormethylpyridin der Formel I, dadurch gekennzeichnet, daß man 2-Amino-4-picolin mit Phthalsäureanhydrid zu 4-Methyl-2-phthalimido-pyridin umsetzt, dieses durch radikalische Chlorierung in Gegenwart eines Radikalstarters und /oder einer UV-Lichtquelle bei Temperaturen von 50 bis 250°C in einem für die Halogenierung geeigneten Lösungsmittel in das 4-Trichlormethyl-2-phthalimido-pyridin überführt und das 2-Amino-4-trichlormethylpyridin hieraus durch Verseifung in an sich bekannter Weise freisetzt.

3. Verfahren nach Anspruch 2, dadurch gekennzeichnet, daß man die radikalische Chlorierung bei Temperaturen von 120 bis 160°C vornimmt.

4. Verfahren nach Anspruch 2, dadurch gekennzeichnet, daß man 0,1 bis 10 Gew.-% eines Radikalbildners, bezogen auf die zu chlorierende Verbindung, zusetzt.

5. Verfahren nach Anspruch 2, dadurch gekennzeichnet, daß man die radikalische Chlorierung in Chlorbenzol, Dichlorbenzol oder Nitrobenzol als Lösungsmittel durchführt.

6. Nitrifikationshemmendes Mittel, enthaltend eine wirksame Menge 2-Amino-4-trichlormethylpyridin.

**Claims**

1. 2-Amino-4-trichloromethylpyridine of the formula I

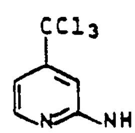

(I)

2. A process for the preparation of 2-amino-4-trichloromethylpyridine of the formula I, wherein 2-amino-4-picoline is reacted with phthalic anhydride to give 4-methyl-2-phthalimidopyridine, and the latter is converted by free radical chlorination in the presence of a free radical initiator and/or of a UV light source at from 50 to 250°C, in a solvent suitable for halogenation, into 4-trichloromethyl-2-phthalimidopyridine, and 2-amino-4-trichloromethylpyridine is liberated therefrom by hydrolysis in a conventional manner.

3. A process as claimed in claim 2, wherein the free radical chlorination is carried out at from 120 to 160°C.

4. A process as claimed in claim 2, wherein from 0.1 to 10 % by weight, based on the compound to be chlorinated, of a free radical initiator are added.

5. A process as claimed in claim 2, wherein the free radical chlorination is carried out in chlorobenzene, dichlorobenzene or nitrobenzene as the solvent.

6. A nitrification inhibitor containing an effective amount of 2-amino-4-trichloromethylpyridine.

**Revendications**

1. 2-amino-4-trichlorométhylpyridine de formule I

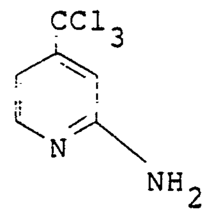

(I)

2. Procédé de préparation de 2-amino-4-trichlorométhylpyridine de formule I, caractérisé par le fait que l'on fait réagir de la 2-amino-4-picoline avec de l'anhydide phtalique pour obtenir de la 4-méthyl-2-phtalimido-pyridine, on transforme celle-ci, par chloration radicalaire, en présence d'un starter de radical et/ou une source de lumière UV, à des températures de 50 a 250° C, dans un solvant approprié pour l'halogénation, en la 4-trichlorométhyl-2-phtalimido-pyridine, et on libère de celle-ci la 2-amino-4-trichlorométhylpyridine par saponification, de manière connue en soi.

3. Procédé selon la revendication 2, caractérisé par le fait que l'on effectue la chloration radicalaire à des températures de 120 à 160° C.

4. Procédé selon la revendication 2, caractérisé par le fait que l'on ajoute 0,1 à 10 % en poids d'un générateur de radical, rapporté au compose à chlorer.

5. Procédé selon la revendication 2, caractérisé par le fait que l'on effectue la chloration radicalaire dans du chlorobenzène, dichlorobenzène ou nitrobenzène, comme solvant.

6. Agent inhibiteur de nitrification, contenant une quantité efficace de 2-amino-4-trichlorométhylpyridine.